# EUROPEAN PATENT SPECIFICATION

(11) **EP 4 378 449 B1**
(45) Date of publication and mention of the grant of the patent: **29.07.2026**
(21) Application number: 22861490.5
(22) Date of filing: 05.03.2022
(51) Int. Cl.: A61K 8/9794, A61K 8/39, A61K 8/86, A61K 8/34, A61K 8/03, A61Q 19/00, A61Q 19/08, A61Q 19/02, A61K 8/14

(54) **COSMETIC COMPOSITION CONTAINING IRIS GERMANICA ROOT-DERIVED EXOSOMES AS ACTIVE INGREDIENT**
KOSMETISCHE ZUSAMMENSETZUNG MIT EXOSOMEN AUS DER IRISGERMANICAWURZEL ALS WIRKSTOFF
COMPOSITION COSMÉTIQUE CONTENANT DES EXOSOMES DÉRIVÉS DE RACINE D'IRIS GERMANICA EN TANT QUE PRINCIPE ACTIF

(30) Priority: 27.08.2021 KR 20210114181
(43) Date of publication of application: 05.06.2024
(73) Proprietor: PP Produits Prestiges SA, Montreux 1820 (CH); Shinsegae International Inc., Seoul 06015 (KR)
(72) Inventor: KIM, Jeenie, 1820 Montreux (CH); KIM, Young Seok, Seoul 06015 (KR); KIM, Jun Oh, Yongin-si Gyeonggi-do 17005 (KR)
(74) Representative: Isarpatent
(86) International application number: PCT/KR2022/003135
(87) International publication number: WO 2023/027274

(56) References cited:
- EP-A1- 4 342 448
- EP-A2- 0 815 838
- WO-A1-2023/022414
- JP-A- 2010 111 602
- KR-A- 19980 079 285
- KR-A- 20000 075 870
- KR-A- 20170 136 956
- KR-B1- 102 265 811
- KR-B1- 102 348 510
- US-B1- 6 471 997

## Description

### [Technical Field]

The present invention relates to a cosmetic composition containing iris rhizome-derived exosomes as an active ingredient. Specifically, the present invention is directed to a cosmetic composition containing as an active ingredient iris rhizome-derived exosomes purified using a pretreatment of ultra-high pressure and an aqueous two-phase system, thereby having excellent stability as well as excellent skin moisturizing, skin wrinkle improvement, and skin whitening effect.

### [Background Art]

In the present invention, "exosomes" refer to small vesicles with a membrane structure secreted from various cells and is defined as a type of extracellular vesicles (EVs). All cells exchange information with other cells or an external environment and secrete the extracellular vesicles for this exchange. The exosomes have a size of about 50 to 200 nm and contain a biologically active substance such as a protein, a lipid, and a nucleic acid. The Exosomes exist in various cells such as a mammal, bacteria and a plant, and can be used for diagnosis and treatment because it reflects a condition of the originating cells. The exosomes are double phospholipid membrane structures that easily penetrate the cells and perform various physiological and pathological functions such as immune response and signal transfer.

Recently, research has been conducted on various effects of the exosomes derived from plants, and has also begun on their skin efficacy such as antioxidant property and anti-inflammatory property. The plant-derived exosomes are natural nanoparticles that contain physiological activity and signal transfer substances secreted by the plant cells themselves and help to move and absorb between the cells. The exosomes purified from the plant have been known to be less toxic than mammalian-derived exosomes.

In this connection, Korean Patent Registration No. 10-2125567 discloses a method of extracting a plant exosome of high purity from a raw plant using a centrifugation and a tangential-flow filtration (TFF). Korean Patent Laid-open Publication. No. 10-2020-0121062 discloses a method for purifying extracellular vesicles of high purity and high quality using a size exclusion chromatography, and Korean Patent Laid-open Publication No. 10-2019-0050286 discloses a filler composition having improved stability of exosomes by adding the exosomes into a hyaluronic acid-based filler composition.

US6471997 B1 discloses an extract of cells of Iridaceae such as Iris, particularly Iris pallida, whereby the extract may be made from the root, ie. rhizome. Example 1 shows the preparation of such an extract whereby Iris pallida cells are ground and centrifuged, and the supernatant filtered and kept. Due to the method of extraction, this extract will comprise exosomes.

EP815838 A2 / KR 1998 0079285 A discloses a cosmetic composition comprising a unicellularised plant, such as Iris rhizome.

These exosomes are materials that can be utilized in the field such as a medicine, cosmetics, and a food by virtue of their various advantages and activities, but have a low dispersion and tend to aggregate due to their structural characteristics consisting of a double phospholipid membrane. In addition, they are unstable at a high temperatures and may easily break in the course of preparing a cosmetic formulation, thereby being likely to reduce a stability of the exosomes in the formulation and cause precipitation. Therefore, in order to maintain continuous activity, it is necessary to enhance the stability of the exosome in the formulation by increasing solubility and dispersibility of the exosomes in an aqueous solution.

The present inventors have conducted research for developing plant-derived exosomes with excellent skin efficacy as cosmetics by isolating and purifying various plant-derived exosomes using an aqueous two-phase system, and as a result, have completed the present invention.

### [Disclosure]

### [Technical Problem]

A purpose of the present invention is to provide a cosmetic composition containing iris rhizome-derived exosomes as an active ingredient, thereby having excellent stability as well as excellent skin moisturizing, skin wrinkle improvement, and skin whitening effect.

### [Technical Solution]

In order to achieve the above purpose, according to the present invention, a cosmetic composition containing iris rhizome-derived exosomes is provided.

The iris rhizome-derived exosomes are purified by a method of comprising the steps of: (A) treating iris rhizomes with ultra-high pressure; (B) juicing the iris rhizomes treated with the ultra-high pressure; (C) obtaining a supernatant by centrifuging a juice of the iris rhizomes at 1,000xg to 10,000xg; (D) freeze-drying the supernatant containing exosomes; (E) forming an aqueous two-phase system by applying PEG (polyethylene glycol)/dextran to the freeze-dried product; and (F) obtaining a lower layer solution in which the exosomes are concentrated under the aqueous two-phase system.

A treatment of ultra-high pressure is characterized in that it is performed with a pressure of 200 to 500 MPa at a temperature of 15 to 25 °C for 20 seconds to 2 minutes.

The iris rhizome-derived exosomes as an active ingredient are contained in an amount of 0.0001 to 30.0 % (w/w) based on the total weight of a cosmetic composition.

The cosmetic composition is characterized by further containing at least one selected from the group consisting of polyethylene glycol, polypropylene glycol, a copolymer of polyethylene glycol and polypropylene glycol or a derivative thereof, butylene glycol, propylene glycol, and glycerin so as to enhance a property stability of the iris rhizome exosomes.

The cosmetic composition is characterized by being for moisturizing a skin, improving skin wrinkles, or whitening the skin.

### [Advantage Effects]

The iris rhizome-derived exosomes of the present invention purified using a pretreatment of ultra-high pressure and an aqueous two-phase system have excellent stability and exhibit excellent efficacies of skin moisturizing, skin wrinkle improvement, and skin whitening, so that they can be usefully used as anti-aging cosmetics.

### [Description of the Drawings]

FIG. 1 is a TEM image of iris rhizome-derived exosomes purified according to an embodiment of the present invention.
FIG. 2 is a graph showing the results of NTA analysis to confirm a size distribution and a particle number of iris rhizomes-derived exosome particles purified according to an embodiment of the present invention.
FIG. 3 is a graph showing the results of NTA analysis to confirm a size distribution and a particle number of iris rhizomes-derived exosome particles purified without a pretreatment of ultra-high pressure.
FIG. 4 is a graph showing the results of evaluating a cytotoxicity of iris rhizome-derived exosomes purified according to the present invention by MTT analysis.
FIG. 5 is a graph showing the results of evaluating a moisturizing efficacy of iris rhizome-derived exosomes purified according to the present invention, based on AQP3 expression.
FIG. 6 is a graph showing the results of evaluating a skin wrinkle improvement efficacy of iris rhizome-derived exosomes purified according to the present invention, based on MMP-1 expression.
FIG. 7 is a graph showing the results of evaluating a skin wrinkle improvement efficacy of iris rhizome-derived exosomes purified according to the present invention, based on COL1A1 expression.
FIG. 8 is a graph showing the results of evaluating a whitening efficacy of iris rhizome-derived exosomes purified according to the present invention, based on an inhibition rate of tyrosinase activity.
FIG. 9 is a graph showing the results of evaluating a whitening efficacy of iris rhizome-derived exosomes purified according to the present invention, based on an inhibition rate of melanin production.

### [Mode for Invention]

Hereinafter, the present invention will be described in more detail.

Plant-derived exosomes contain physiological activity and signal transfer substances secreted by the plant cells themselves, and are known to be less toxic than mammalian-derived exosomes. By virtue of these advantages, the plant-derived exosomes are materials that can be utilized in the field such as a medicine, cosmetics, and a food, but since the plant-derived exosomes have a low dispersion and tend to aggregate due to their structural characteristics consisting of a double phospholipid membrane, they have a problem of making it difficult to continuously maintain their activity within a formulation. The technical feature of the present invention is to isolate and purify exosomes from the iris rhizomes with high purity and use them as cosmetics.

An iris is a perennial plant belonging to the family iris and the genus iris. The iris rhizomes have digestive and anti-inflammatory efficacies, so they are used as a medicine for treating indigestion, hemorrhoids, bruises, skin diseases, sore throat, tonsillitis, etc. The iris rhizomes contain a large amount of the fatty acid such as a linoleic acid, a myristic acid, and a lauric acid. An iris germanica, one type of the iris, is a multi-year herb native to Europe and is a perennial plant of the monocotyledonous plant lily tree iris family that is planted and grown in a dry place. The iris germanica is very resistant to cold, so it can overwinter in the open air, and the largest flower among the irises grows in clusters at an end of the flower stem. Since the iris germanica has thick underground stems, it is also treated as a bulbous plant. The iris germanica rhizomes contain isoflavone such as irisolone and irigenin.

The iris rhizome-derived exosomes of the present invention are purified by a method of comprising the steps of:

(A) treating iris rhizomes with ultra-high pressure; (B) juicing the iris rhizomes treated with the ultra-high pressure; (C) obtaining a supernatant by centrifuging a juice of the iris rhizomes at 1,000xg to 10,000xg; (D) freeze-drying the supernatant containing exosomes; (E) forming an aqueous two-phase system by applying PEG (polyethylene glycol)/dextran to the freeze-dried product; and (F) obtaining a lower layer solution in which the exosomes are concentrated under the aqueous two-phase system.

In the step (A), both living and dried iris rhizomes may be used, and the treatment of ultra-high pressure is characterized by being performed with a pressure of 200 Mpa or more, preferably a pressure of 200 to 500 Mpa, at a temperature of 15 to 25 °C for 20 seconds to 2 minutes. The exosomes are a biologically active substance, and it is preferrable to treat them a temperature between 15 °C and 25 °C.

According to an embodiment of the present invention, the treatment of ultra-high pressure is performed by putting the dried iris rhizomes together with a distilled water in a plastic pack and sealing it well to prevent air from entering, and then treating the sealed pack with an ultra-high pressure of 200 MPa or more at a temperature of 15 to 25 °C for 20 seconds to 2 minutes, using an ultra-high pressure machine. In case iris rhizome-derived organisms are used, the treatment of ultra-high pressure may performed by putting them in the plastic pack without the distilled water.

It is preferable that a screw used in the process of juicing the iris rhizomes in the step (B) has a stirring speed of 20 to 50 rpm. Because the juice easily leaks out due to a change in permeability of plant cell walls caused by the treatment of ultra-high pressure, it is recommended to use a screw with a stirring speed below a certain level.

The centrifugation process used in the step (C) refers to a process of separating particles in a solution using a centrifugal force depending on a size, a shape, a density, a viscosity, and a rotor speed. It is necessary to remove large contaminants by sequentially adjusting a rpm, and it is more preferable to perform the process at 10,000xg to obtain a final solution for forming the aqueous two-phase system.

The freeze drying used in the step (D) is a method of rapidly lowering a temperature of a container to freeze materials to be dried followed by directly sublimating a solidified solvent contained in the materials into water vapor, and drying the materials by bringing a pressure inside the container close to a vacuum. The freezing is carried out at -50 to -80 °C for 15 to 24 hours and the drying is performed in a freeze dryer under a vacuum for 72 to 120 hours. In this case, the vacuum state usually refers to a pressure state of the freeze dryer.

In order to efficiently separate and purify the exosomes, the present invention utilized an aqueous two-phase partition method, which is one of the separation methods using a difference in affinity for each layer by creating two layers from two types of aqueous solutions that do not dissolve well with each other.

In general, PEG/salt (sulfate, phosphate, citrate, etc.) may be used in the step of forming the aqueous two-phase system, but it is preferable to use PEG/dextran to achieve the purpose of the present invention. The dextran is a natural polymer obtained through bacterial action and is used as a thickener, a binder, and a bulking agent in a cosmetic formulation.

In the step (E) of forming the aqueous two-phase system, it is characterized in that the PEG having a molecular weight of 10,000 to 35,000 is used in an amount of 1 to 15 % by weight, preferably 2 to 5 % by weight, and the dextran having a molecular weight of 300,000 to 650,000 is used in an amount of 1 to 8 % by weight, preferably 1 to 3 % by weight. It is more preferable to use the PEG and the dextran at a concentration ratio of 3.3 % by weight: 1.7 % by weight, because a yield of the exosomes is highest and stability is the best.

In order to increase a purity of the exosomes, following the step (F), a process of forming the aqueous two-phase system using an aqueous two-phase solution of the same concentration and obtaining a lower layer solution may be additionally performed two to three times.

The iris rhizome-derived exosomes prepared by such a method indicated excellent moisturizing effect (Test Example 4), excellent wrinkle improvement effect (Test Example 5, Test Example 6, Test Example 9), and excellent whitening effect (Test Example 7, Test Example 8), compared to iris rhizome extracts. In addition, the iris rhizome-derived exosomes had excellent stability within the formulation (Test Examples 9 and 10).

Therefore, the iris rhizome-derived exosomes can be used in a cosmetic composition for moisturizing, wrinkle improvement, or whitening. In this case, the iris rhizome-derived exosomes as the active ingredient may be contained in an amount of 0.0001 to 30.0 % (w/w) based on the total weight of the cosmetic composition.

The cosmetic composition is characterized by containing preferably at least one selected from the group consisting of polyethylene glycol, polypropylene glycol, a copolymer of polyethylene glycol and polypropylene glycol or a derivative thereof, butylene glycol, propylene glycol, and glycerin, more preferably at least one selected from the group consisting of glycerin, butylene glycol, and 1,3-propylene glycol, most preferably at least one selected from the group consisting of butylene glycol and 1,3-propylene glycol, so as to increase a property stability of the iris rhizome-derived exosomes.

In general, the exosomes have a low dispersion and tend to aggregate due to their structural characteristics of consisting of a double phospholipid membrane, thereby having a problem of making it difficult to continuously maintain their activity within the formulation. However, in case the iris rhizome-derived exosomes are used together with a compound containing an alcohol group, selected from the group consisting of polyethylene glycol, polypropylene glycol, a copolymer of polyethylene glycol and polypropylene glycol or a derivative thereof, butylene glycol, propylene glycol, and glycerin, they had more excellent stability.

The cosmetic composition can be used even in any formulation commonly prepared, and can be formulated, for example, into a skin lotion, a skin softener, a skin toner, a lotion, a milk lotion, a moisture lotion, a nutritional lotion, a massage cream, a nutritional cream, a moisture cream, a hand cream, an essence, a pack, a soap, a shampoo, a cleansing foam, a cleansing lotion, a cleansing cream, a body lotion, a body cleanser, an emulsion, a press powder, a loose powder, etc.

On the other hand, in case the iris rhizome-derived exosomes are prepared into the above cosmetic composition, components commonly used in the cosmetics, for example, conventional auxiliaries such as a moisturizer, an antioxidant, a surfactant, alcohol, a thickener, an aqueous ingredient, water, an emulsifier, a vitamin, a pigment, and a fragrance may be appropriately selected and added within the boundary that does not impair the effect of the present invention by a person skilled in the art without difficulty, if necessary.

### [Example]

Hereinafter, the present invention will be described in more detail based on the following Examples and Test Examples. However, since the following Examples are only for illustrating the present invention, the present invention is not limited by the following Examples. It will be obvious by a person who has an ordinary skill in the technical field to which the present invention pertains that substitution and other equivalent modification for Examples may be made without departing from the technical spirit of the present invention.

### Example 1: Preparation of iris germanica rhizome-derived exosomes

### Treatment of ultra-high pressure of iris germanica rhizomes

100 g of dried iris germanica rhizomes were putted into a plastic pack together with distilled water and sealed well to prevent air from entering, and then subjected to a treatment of ultra-high pressure with a pressure of 200 MPa at a temperature of 25 °C for 30 seconds using an ultra-high pressure machine.

### Juicing of iris germanica rhizomes

The iris germanica rhizomes treated with the ultra-high pressure were juiced with a low-speed screw of 30 rpm using a regular juicer, and the obtained juice of iris germanica rhizomes was filtered through a mesh net to remove suspended substances. The recovered juice of iris germanica rhizomes was stored at -80 °C until purification.

### Recovery of supernatant for exosome purification

Because the juice of iris germanica rhizomes requires removal of large contaminants for exosome purification, centrifugation was performed at 10,000xg at 4 °C for 10 minutes. After the centrifugation, a supernatant was recovered to form an aqueous two-phase system.

### Freeze-drying of supernatant

For mass production of the exosomes, freeze-drying was performed to reduce a volume of the supernatant. The supernatant was frozen at -80 °C for 20 hours and dried in a freeze dryer under a vacuum for 100 hours. In this case, the vacuum state usually refers to a pressure state of the freeze dryer, and the freezing and drying time may vary depending on a volume of the solution.

### Formation of aqueous two-phase system

Purified water was added to the freeze-dried supernatant, and an aqueous two-phase system was formed using PEG (polyethylene glycol)/dextran. The PEG (purchased from Sigma Aldrich) with a molecular weight of 10,000 to 35,000 was used in an amount of 3.3 % by weight and the dextran (purchased from Sigma Aldrich) with a molecular weight of 300,000 to 650,000 was used in an amount of 1.7 % by weight to form the aqueous two-phase system.

### Recovery of iris germanica rhizome-derived exosomes

After mixing the supernatant and the PEG/dextran solution, centrifugation was performed at 1,000xg at 4 °C for 10 minutes. After the centrifugation, the supernatant was removed to recover exosomes.

### Additional washing process

in order to increase a purity of the exosomes, an additional washing process was performed by adding the same concentration of the aqueous two-phase solution to a lower layer of the recovered solution. After repeated processing three times, the lower layer having final exosomes concentrated was recovered.

### Comparative Example 1: Purification of iris germanica rhizome-derived exosomes without treatment of ultra-high pressure

Iris germanica rhizome-derived exosomes were purified in the same method as that of Example 1, except that the treatment of ultra-high pressure was not performed.

### Comparative Example 2: Preparation of hydrothermal extracts of iris germanica rhizomes

20 g of dried iris germanica rhizomes were putted into 800 g of purified water and extracted at 80 °C for 3 hours. After the extraction, filtration was performed under a reduced pressure to obtain hydrothermal extracts of the iris germanica rhizomes, and then distilled with a rotary evaporation concentrator to obtain a sample in the form of powders.

### Comparative Example 3: Preparation of methanol extracts of iris germanica rhizomes

20 g of dried iris germanica rhizomes were putted into 800 g of methanol and extracted at 60 °C for 3 hours. After the extraction, filtration was performed under a reduced pressure to obtain methanol extracts of the iris germanica rhizomes, which were then distilled with a rotary evaporation concentrator to obtain a sample in the form of powders.

### Comparative Example 4: Preparation of ethanol extracts of iris germanica rhizomes

20 g of dried iris germanica rhizomes were putted into 800 g of ethanol and extracted at 60 °C for 3 hours. After the extraction, filtration was performed under a reduced pressure to obtain ethanol extracts of the iris germanica rhizomes, which were then distilled with a rotary evaporation concentrator to obtain a sample in the form of powders.

### Comparative Example 5: Preparation of hexane extracts of iris germanica rhizomes

20 g of dried iris germanica rhizomes were putted into 800 g of hexane and extracted at 60 °C for 3 hours. After the extraction, filtration was performed under a reduced pressure to obtain hexane extracts of the iris germanica rhizomes, which were then distilled with a rotary evaporation concentrator to obtain a sample in the form of powders. Table 1 below shows an extraction yield for each solvent.

**[Table 1]**

| | Comparative Example 2 | Comparative Example 3 | Comparative Example 4 | Comparative Example 5 |
|---|---|---|---|---|
| Extracted solvent | Purified water | Methanol | Ethanol | Hexane |
| Weight of powders (g) | 3.32 | 2.95 | 2.41 | 2.44 |
| Yield (%) | 16.60 | 14.75 | 12.05 | 12.20 |

### Test Example 1: Characteristic analysis of iris germanica rhizome-derived exosomes : TEM analysis

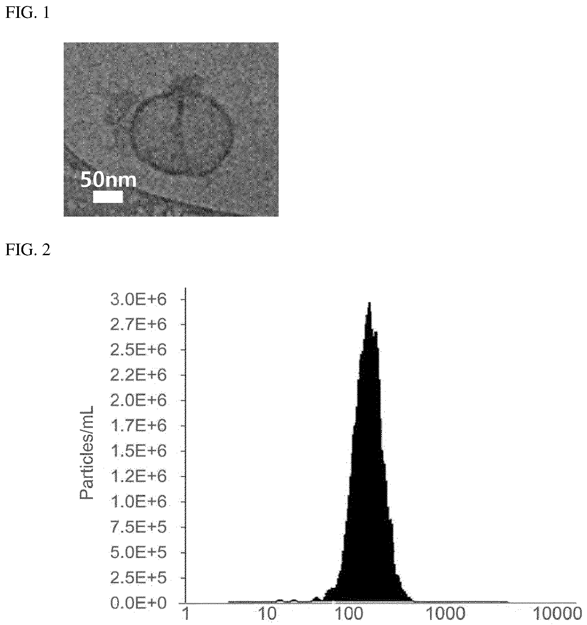

In order to confirm a shape of the purified iris germanica rhizome-derived exosomes, they were analyzed by a transmission electron microscopy (TEM). FIG. 1 is a TEM analysis image of the iris germanica rhizome-derived exosomes purified according to Example 1. As a result of the analysis, particles of approximately 160.7 nm having a spherical structure of double phospholipid layers were confirmed to exist.

### Test Example 2: Characteristic analysis of iris germanica rhizome-derived exosomes : NTA analysis

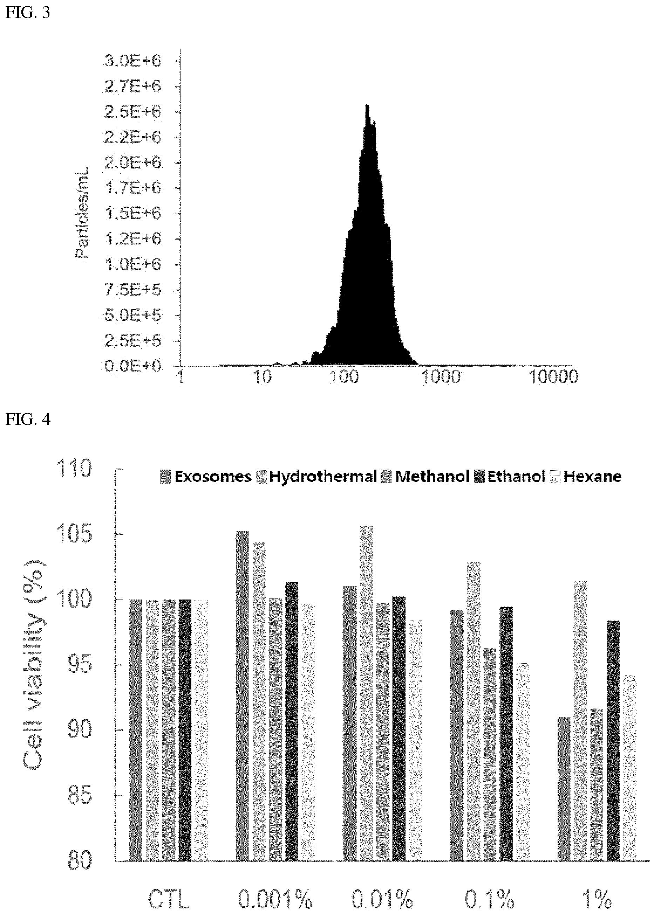

The purified iris germanica rhizome-derived exosomes were analyzed with a nanoparticle tracking analysis (NTA) to confirm a particle size distribution and number of particles per unit volume thereof. In order to compare the number of exosome particles depending on whether the treatment of ultra-high pressure is performed or not, the NTA analysis of the iris germanica rhizome-derived exosomes purified according to Comparative Example 1 was also performed. FIG. 2 is a graph showing the results of NTA analysis of the iris germanica rhizome-derived exosomes purified according to Example 1. As a result of the analysis, it was confirmed that the particles had an average size of 160.7 nm and a concentration of 9.90 x 10⁹ per 1 mL of unit volume. FIG. 3 is a graph showing the results of NTA analysis of the iris germanica rhizome-derived exosomes purified according to Comparative Example 1, which confirmed that the particles had an average size of 172.1 nm and a concentration of 1.61 x 10⁹ per 1 mL of unit volume. Example and Comparative Example 1 were tested with the same amount, and when comparing yields thereof, it was confirmed that a larger amount of the exosomes could be extracted when the treatment of ultra-high pressure was performed (Example).

### Test Example 3: Evaluation of cytotoxicity of iris germanica rhizome-derived exosomes

A MTT assay evaluation was performed to confirm a cytotoxicity of the iris germanica rhizome-derived exosomes (Example 1) and the iris germanica rhizome extracts for each solvent (Comparative Examples 2, 3, and 4). A human dermal fibroblast (HDFa) was inoculated in a 96 well plate at a concentration of 1×10⁵ cells/mL, and then cultured under 5 % CO₂ at 37 °C for 18 hours. After the culture, the medium was removed and washed with a PBS buffer, and then the iris germanica rhizome-derived exosomes (stock concentration 9.90×10⁹ particles/mL) and the iris germanica rhizome extracts for each solvent (stock concentration 100 mg/mL) were administered into a new medium at each concentration and cultured again for 48 hours. In order to measure cell viability, formazan formed over 4 hours after adding a MTT solution (5 mg/mL) was dissolved with dimethyl sulfoxide (DMSO) to measure an absorbance at 570 nm using an ELISA reader. FIG. 4 is a graph showing the results of evaluating a cytotoxicity of the iris germanica rhizome-derived exosomes purified according to Example 1 by the MTT analysis. As a result of the test, it was found that the cytotoxicity was not observed when the stock of the iris germanica rhizome-derived exosomes having a concentration of 9.90×10⁹ particles/mL was treated with 0.1 % or less, and the cell viability was about 90 % when it was treated with 1 % or more.

### Test Example 4: Evaluation of moisturizing efficacy of iris germanica rhizome-derived exosomes (AQP3 expression)

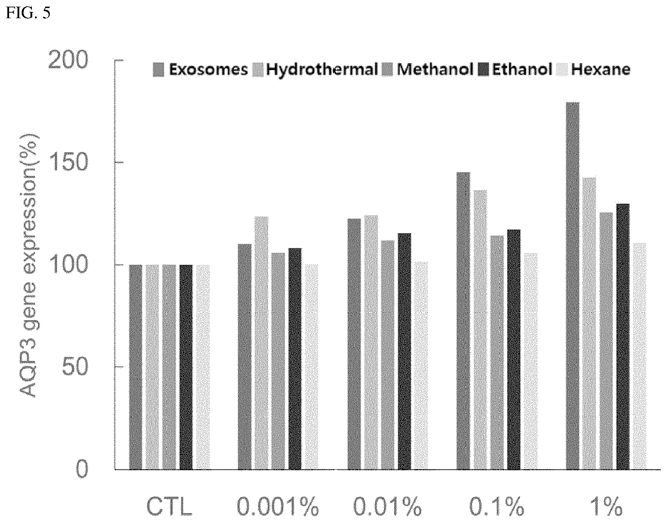

In order to confirm a moisturizing efficacy of the iris germanica rhizome-derived exosomes, they were evaluated for their effect on AQP3 expression by comparing them with the iris germanica rhizome extracts for each solvent. After inoculating a human epidermal keratinocyte (HEKa), they were cultured in a culture medium of Dulbecco Modified Eagle Medium (DMEM) supplemented with 100 IU/mL of penicillin and 100 µg/mL of streptomycin under 5% CO₂ at 37 °C for 24 hours. After the culture, the medium was discarded, and the iris germanica rhizome-derived exosomes (stock concentration 9.90×10⁹ particles/mL) and the iris germanica rhizome extracts for each solvent (stock concentration 100 mg/mL) were treated at each concentration and cultured for 48 hours. Thereafter, RNA was extracted from the cultured cells using a TransZol reagent, followed by performing RT-PCR (real-time genetic polymerase chain reaction) to measure an amount of mRNA change in AQP3 (Aquaporin 3), which is a moisturizing factor involved in moisture movement in the cells and is a protein that plays an important role in skin moisturization. A product generated by the PCR was identified with a Gel Documentation system by performing electrophoresis on a 1% agarose gel. In this case, a negative control group was treated with PBS, and a positive control group was treated with 0.01 % of a hyaluronic acid. FIG. 5 is a graph showing the results of evaluating a moisturizing efficacy of the iris germanica rhizome-derived exosomes purified according to Example, based on the AQP3 expression. As a result of the test, it was confirmed that the mRNA expression of AQP3 increased in a concentration-dependent manner when treated with the iris germanica rhizome-derived exosomes and the iris germanica rhizome extracts, and that the expression further increased when treated with the iris germanica rhizome-derived exosomes. In this case, an expression rate was found to be 155.69 % when treated with 0.01% of a hyaluronic acid, a positive control group.

### Test Example 5: Evaluation of wrinkle improvement efficacy of iris germanica rhizome-derived exosomes (MMP-1 expression)

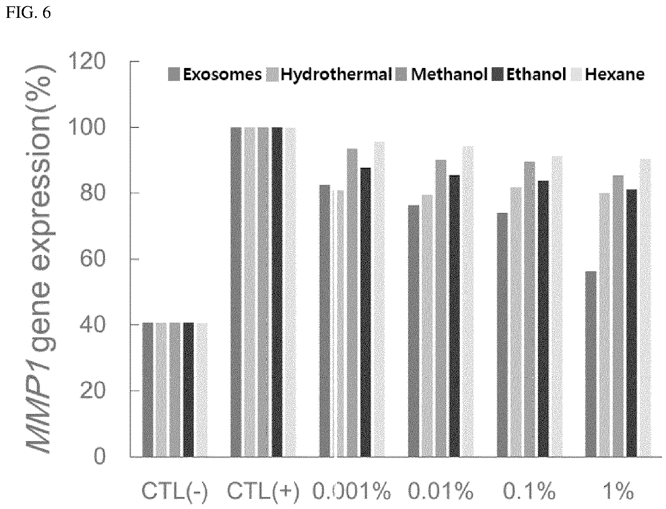

In order to confirm an anti-aging efficacy of the iris germanica rhizome-derived exosomes, they were evaluated for their effect on MMP-1 expression by comparing them with the iris germanica rhizome extracts for each solvent. After inoculating a human dermal fibroblast (HDFa), they were cultured in a culture medium of Fibroblast Basal Medium (Medium 106) containing 100 IU/mL of penicillin and 100 µg/mL of streptomycin under 5% CO₂ at 37 °C for 24 hours. After the culture, the medium was discarded, and the iris germanica rhizome-derived exosomes (stock concentration 9.90×10⁹ particles/mL) and the iris germanica rhizome extracts for each solvent (stock concentration 100 mg/mL) were treated at each concentration and cultured for 48 hours. In order to induce cell damage, the remaining cultured cell groups except for the non-UVB irradiation group were removed from the medium, and then the medium was irradiated with 20 mJ/cm² of UVB and replaced with a new medium, which was then cultured for 24 hours. After 24 hours, RNA was extracted from the cultured cells using a TransZol reagent, followed by performing RT-PCR (real-time genetic polymerase chain reaction) to measure an amount of mRNA change in MMP-1, which is related to skin aging and wrinkle formation. A product generated by the PCR was identified with a Gel Documentation system by performing electrophoresis on a 1% agarose gel. In this case, a negative control group was treated with PBS, and a positive control group was treated with 50 µM of retinyl palmitate. FIG. 6 is a graph showing the results of evaluating the anti-aging efficacy of the iris germanica rhizome-derived exosomes purified according to Example, based on the MMP-1 expression. As a result of the test, it was confirmed that the MMP-1 expression decreased in a concentration-dependent manner when treated with the iris germanica rhizome-derived exosomes and the iris germanica rhizome extracts for each solvent, and that the expression showed much higher decrease when treated with the iris germanica rhizome-derived exosomes. In this case, an expression rate was found to be 72.81 % when treated with 50 µM of retinyl palmitate, a positive control group.

### Test Example 6: Evaluation of wrinkle improvement efficacy of iris germanica rhizome-derived exosomes (COL1A1 expression)

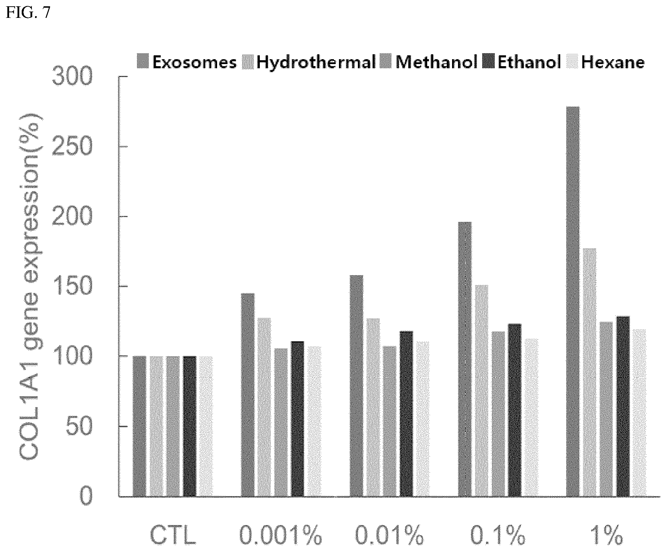

In order to confirm an anti-aging efficacy of the iris germanica rhizome-derived exosomes, they were evaluated for their effect on COL1A1 expression by comparing them with the iris germanica rhizome extracts for each solvent. After inoculating a human dermal fibroblast (HDFa), they were cultured in a culture medium of Fibroblast Basal Medium (Medium 106) containing 100 IU/mL of penicillin and 100 µg/mL of streptomycin under 5% CO₂ at 37 °C for 24 hours. After culturing, the medium was discarded, and the iris germanica rhizome-derived exosomes (stock concentration 9.90×10⁹ particles/mL) and the iris germanica rhizome extracts for each solvent (stock concentration 100 mg/mL) were treated at each concentration and cultured for 48 hours. After culturing, RNA was extracted from the cultured cells using a TransZol reagent, followed by performing RT-PCR (real-time genetic polymerase chain reaction) to measure an amount of mRNA change in collagen, which maintains a skin connective tissue. A product generated by the PCR was identified with a Gel Documentation system by performing electrophoresis on a 1% agarose gel. In this case, a negative control group was treated with PBS, and a positive control group was treated with 50 µM of retinyl palmitate. FIG. 7 is a graph showing the results of evaluating the anti-aging efficacy of the iris germanica rhizome-derived exosomes purified according to Example, based on the COL1A1 expression. As a result of the test, it was confirmed that the COL1A1 expression increased in a concentration-dependent manner when treated with the iris germanica rhizome-derived exosomes and the iris germanica rhizome extracts, and that the expression further increased when treated with the iris germanica rhizome-derived exosomes. In this case, an expression rate was found to be 173.93 % when treated with 50 µM of retinyl palmitate, a positive control group.

### Test Example 7: Evaluation of whitening efficacy of iris germanica rhizome-derived exosomes (inhibition of tyrosinase activity)

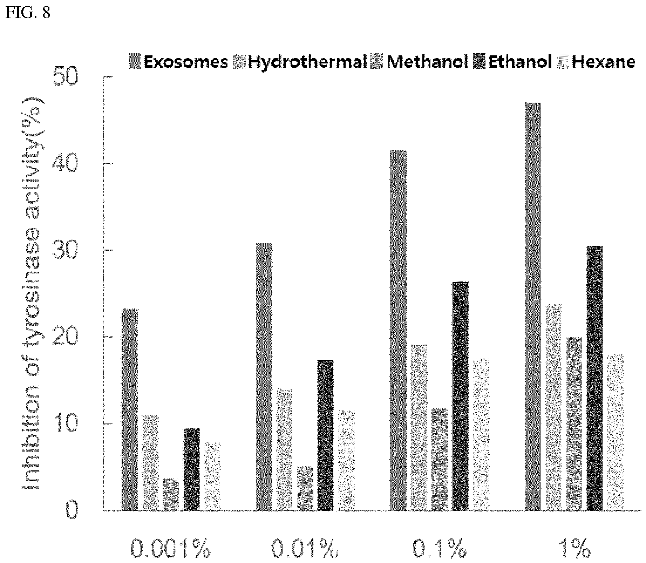

In order to confirm a whitening efficacy of the iris germanica rhizome-derived exosomes, an inhibition test of tyrosinase activity for them was conducted by comparing them with the iris germanica rhizome extracts for each solvent. Mushroom-derived tyrosinase and tyrosine were purchased and used from Sigma Chemical. The tyrosinase activity was evaluated by treating the iris germanica rhizome-derived exosomes (stock concentration 9.90×10⁹ particles/mL) and the iris germanica rhizome extracts for each solvent (stock concentration 100 mg/mL) together with 150 µl of 0.1M phosphate buffer (pH 6.5) and 8 µl of mushroom tyrosinase (2100 units/ml, 0.05M phosphate buffer, pH 6.5), and 36 µℓ of L-tyrosine having a concentration of 1.5 mM, at each concentration. Inhibition of the tyrosinase activity was evaluated by reacting the samples at 37 °C for 15 minutes followed by measuring an absorbance at 490 nm. In this case, a negative control group was treated with PBS, and arbutin, a synthetic substance known as a whitening agent, was used as a standard sample and the results were converted to %. FIG. 8 is a graph showing the results of evaluating the whitening efficacy of the iris germanica rhizome-derived exosomes purified according to Example, based on an inhibition rate of tyrosinase activity. As a result of the test, it was confirmed that the inhibition rate of tyrosinase activity increased in a concentration-dependent manner when treated with the iris germanica rhizome-derived exosomes and the iris germanica rhizome extracts, and that the inhibition rate showed higher increase when treated with the iris germanica rhizome-derived exosomes.

### Test Example 8: Evaluation of whitening efficacy of iris germanica rhizome-derived exosomes (inhibition of melanin production)

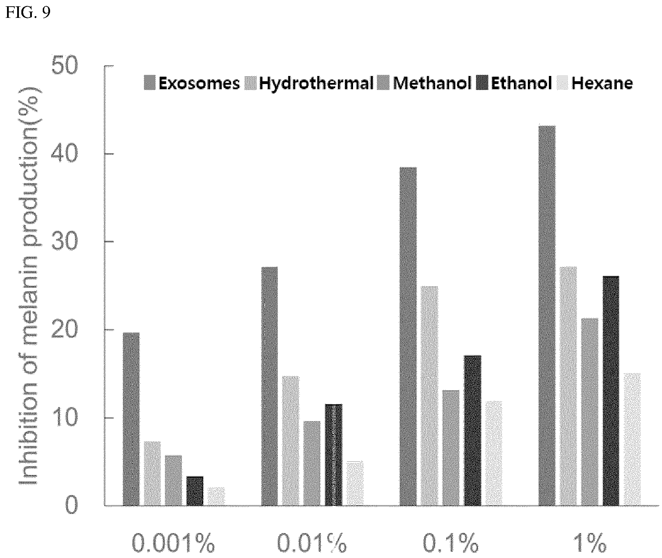

In order to confirm a whitening efficacy of the iris germanica rhizome-derived exosomes, an inhibition test of melanin production for them was conducted by comparing them with the iris germanica rhizome extracts for each solvent. After inoculating a mouse-derived B16F10 cell line (melanin-secreting cells), the cells were cultured in a culture medium of Dulbecco Modified Eagle Medium (DMEM) supplemented with 10 % FBS under 5% CO₂ at 37 °C for 24 hours. After culturing, the medium was discarded and replaced with a new medium, and then the cells were treated with the iris germanica rhizome-derived exosomes (stock concentration 9.90×10⁹ particles/mL) and the iris germanica rhizome extracts for each solvent (stock concentration 100 mg/mL) and cultured for 72 hours. After culturing, the cells were treated with Trypsin-EDTA and recovered by centrifugation. The recovered cells were washed, treated with 500 µL of 1N NaOH, and reacted at 100 °C for 10 minutes to dissolve a melanin. An amount of the melanin was identified by measuring an absorbance at 405 nm. In this case, a negative control group was treated with PBS, and arbutin, a synthetic substance known as a whitening agent, was used as a standard sample and the results were converted to %. FIG. 9 is a graph showing the results of evaluating the whitening efficacy of the iris germanica rhizome-derived exosomes purified according to Example, based on an inhibition rate of melanin production. As a result of the test, it was confirmed that the inhibition rate of melanin production increased in a concentration-dependent manner when treated with the iris germanica rhizome-derived exosomes and the iris germanica rhizome extracts, and that the inhibition rate showed higher increase when treated with the iris germanica rhizome-derived exosomes.

### Formulation Example 1: Preparation of water-typed formulation

A water-typed formulation containing the iris germanica rhizome-derived exosomes purified according to Example was prepared with a composition shown in Table 2 below. In order to further increase a stability of the exosomes purified by an aqueous two-phase system in the formulation, the formulation was prepared by adding to the iris germanica rhizome-derived exosomes at least one compound containing an alcohol group selected from the group consisting of polyethylene glycol, polypropylene glycol, a copolymer of polyethylene glycol and polypropylene glycol or a derivative thereof, butylene glycol, propylene glycol, and glycerin. The formulation that did not contain the above compound was designated as Comparative Formulation Example 1.

**[Table 2]**

| Components | Formulation Example 1 | Comparative Formulation Example 1 |
|---|---|---|
| | Content (% by weight) | |
| Iris germanica rhizome-derived exosomes (Example 1) | 10 | 10 |
| Butylene glycol | 10 | - |
| Propanediol (1,3-propylene glycol) | 10 | - |
| 1,2-hexanediol | 2 | 2 |
| Purified water | 68 | 88 |

### Test Example 9: Stability analysis at each temperature of water-typed formulation containing iris germanica rhizome-derived exosomes

A stability test was conducted at each temperature of the water-typed formulations prepared according to Formulation Example 1 and Comparative Formulation Example 1. Table 3 below shows a size and concentration of particles through a nanoparticle tracking analysis (NTA) after storing the formulation of Formulation Example 1 and the formulation of Comparative Formulation Example 1 at 4 °C, 25 °C, and 45 °C for 12 weeks.

**[Table 3]**

| Formulation Example 1 | Formulation Example 1 (Storing at 4°C) | | Formulation Example 1 (Storing at 25°C) | | Formulation Example 1 (Storing at 45°C) | |
|---|---|---|---|---|---|---|
| | Particle Number (Particles/ml) | Particle Size (nm) | Particle Number (Particles/ml) | Particle Size (nm) | Particle Number (Particles/ml) | Particle Size (nm) |
| Beginning | 9.90x10⁹ | 160.7 | 9.90x10⁹ | 160.7 | 9.90x10⁹ | 160.7 |
| 2^{nd} week | 9.79x10⁹ | 161.5 | 9.71x10⁹ | 163.5 | 9.84x10⁹ | 160.6 |
| 4^{th} week | 9.81x10⁹ | 159.1 | 9.73x10⁹ | 167.1 | 9.70x10⁹ | 157.8 |
| 8^{th} week | 9.67x10⁹ | 157.7 | 9.57x10⁹ | 162.7 | 9.78x10⁹ | 165.7 |
| 12^{th} week | 9.71x10⁹ | 162.9 | 9.62x10⁹ | 159.4 | 9.71x10⁹ | 163.0 |

| Comparative Formulation Example 1 | Comparative Formulation Example 1 (Storing at 4°C) | | Comparative Formulation Example 1 (Storing at 25°C) | | Comparative Formulation Example 1 (Storing at 45°C) | |
|---|---|---|---|---|---|---|
| | Particle Number (Particles/ml) | Particle Size (nm) | Particle Number (Particles/ml) | Particle Size (nm) | Particle Number (Particles/ml) | Particle Size (nm) |
| Beginning | 9.90x10⁹ | 160.7 | 9.90x10⁹ | 160.7 | 9.90x10⁹ | 160.7 |
| 2^{nd} week | 8.41x10⁹ | 162.5 | 8.20x10⁹ | 164.7 | 6.05x10⁹ | 157.2 |
| 4^{th} week | 5.01x10⁹ | 157.1 | 6.49x10⁹ | 160.1 | 4.28x10⁹ | 155.4 |
| 8^{th} week | 2.18x10⁹ | 155.7 | 1.77x10⁹ | 162.6 | 9.89x10⁸ | 157.6 |
| 12^{th} week | 8.90x10⁸ | 156.9 | 9.06x10⁸ | 154.3 | 6.04x10⁸ | 156.1 |

As a result of analyzing the stability at each temperature for 12 weeks, it was confirmed that a change in the size and concentration of the iris germanica rhizome-derived exosomes corresponding to Formulation Example 1 mixed with polyol of Formulation Example 1 was stable at 4 °C, 25 °C, and 45 °C in all. Meanwhile, it was confirmed from Comparative Formulation Example 1 that the concentration of the exosomes decreased at 4 °C, 25 °C, and 45 °C in all.

### Formulation Example 2: Preparation of cream

A cream containing the iris germanica rhizome-derived exosomes purified according to Example was prepared with a composition shown in Table 4 below by a conventional method. When comparing an efficacy of the iris germanica rhizome extracts for each solvent, the cream containing a hydrothermal extract of the iris germanica rhizomes according to Comparative Example 2, which had good efficacy, was used as Comparative Formulation Example 2.

**[Table 4]**

| Components | Formulation Example 2 | Comparative Formulation Example 2 |
|---|---|---|
| | Content (% by weight) | |
| Iris germanica rhizome-derived exosomes (Example 1) | 3 | - |
| Hydrothermal extract of iris germanica rhizomes (Comparative Example 2) | - | 3 |
| Glycerin | 10 | 10 |
| Butylene glycol | 5 | 5 |
| Glyceryl oleate | 1.8 | 1.8 |
| Cetearyl olivate | 0.5 | 0.5 |
| Sorbitan olivate | 0.5 | 0.5 |
| Caprylic/capric triglyceride | 5.0 | 5.0 |
| Cetyl ethylhexanoate | 1.0 | 1.0 |
| Beeswax | 0.5 | 0.5 |
| Squalane | 0.2 | 0.2 |
| 1,2-hexanediol | 0.2 | 0.2 |
| Cholesteryl/behenyl/octyldodecyl lauroyl glutamate | 1.0 | 1.0 |
| Dimethicone | 0.5 | 0.5 |
| Cyclopentasiloxane/Cyclohexasiloxane | 2.0 | 2.0 |
| Cetearyl alcohol | 1.0 | 1.0 |
| Mineral oil | 2.5 | 2.5 |
| Disodium EDTA | 0.02 | 0.02 |
| BHT | 0.05 | 0.05 |
| Tocopheryl acetate | 0.3 | 0.3 |
| Panthenol | 0.2 | 0.2 |
| Ethylhexyl methoxycinnamate | 0.2 | 0.2 |
| Fragrance | 0.01 | 0.01 |
| Purified water | Residual amount | Residual amount |

### Test Example 10: Analysis of property stability of iris germanica rhizome-derived exosome formulation

A property stability test was conducted on the cream prepared according to Formulation Example 2. A change in the property of the prepared cream was observed in a cycling chamber for 12 weeks while subjecting the cream to a temperature change of 4 °C ↔ 45 °C at 24-hour intervals. The results are shown in Table 5 below.

**[Table 5]**

| Week | Formulation Example 2 |
|---|---|
| Beginning | - |
| 2^{nd} week | - |
| 4^{th} week | - |
| 8^{th} week | - |
| 12^{th} week | + |

(-; no change, +; slight change in color, ++; change in color or slight precipitation, +++; change in color and occurrence of precipitation)

As a result of observing the stability of the cream formulation containing the iris germanica rhizome-derived exosomes purified by an aqueous two-phase system (Example), it was confirmed that the property stability of the cream was maintained.

### Test Example 11: Skin wrinkle improvement effect of iris germanica rhizome-derived exosomes

30 adult women in their 30s to 50s were divided into two groups: a cream (Formulation Example 2) containing the iris germanica rhizome-derived exosomes purified according to Example and a cream (Comparative Formulation Example 2) containing the hydrothermal extract of the iris germanica rhizomes according to Comparative Example 2 were applied to both sides of the woman faces and irradiated with a red light of 633 nm for 24 hours cumulatively for 6 weeks, using an LED light source device, and then a wrinkle improvement effect was evaluated. Table 6 below shows results of the wrinkle improvement effect based on this evaluation.

**[Table 6]**

| | Wrinkle improvement effect | | |
|---|---|---|---|
| | Excellent | Little | None |
| Formulation Example 2 | 11 | 3 | 1 |
| Comparative Formulation Example 2 | 4 | 4 | 7 |

As can be seen from the results of Table 6 above, the cream containing the iris germanica rhizome-derived exosomes of the present invention showed an excellent skin wrinkle improvement effect.

## Claims

1. A cosmetic composition comprising iris rhizome-derived exosomes, wherein the iris rhizome-derived exosomes are purified by a method of comprising the steps of:
(A) treating iris rhizomes with ultra-high pressure;
(B) juicing the iris rhizomes treated with the ultra-high pressure;
(C) obtaining a supernatant by centrifuging a juice of the iris rhizomes at 1,000xg to 10,000xg;
(D) freeze-drying the supernatant containing exosomes;
(E) forming an aqueous two-phase system by applying PEG (polyethylene glycol)/dextran to the freeze-dried product; and
(F) obtaining a lower layer solution in which the exosomes are concentrated under the aqueous two-phase system,
wherein the treatment of ultra-high pressure is performed with a pressure of 200 to 500 MPa at a temperature of 15 to 25 °C for 20 seconds to 2 minutes,
wherein the cosmetic composition further contains at least one selected from the group consisting of polyethylene glycol, polypropylene glycol, a copolymer of polyethylene glycol and polypropylene glycol or a derivative thereof, butylene glycol, propylene glycol, and glycerin, so as to enhance a property stability of the iris rhizome-derived exosomes.

2. The cosmetic composition according to claim 1, **characterized in that** the iris rhizome-derived exosomes are comprised in an amount of 0.0001 to 30.0 % (w/w) based on the total weight of the cosmetic composition.

3. The cosmetic composition according to claim 1, **characterized by** being for moisturizing a skin.

4. The cosmetic composition according to claim 1, **characterized by** being for improving skin wrinkles.

5. The cosmetic composition according to claim 1, **characterized by** being for whitening a skin.

## Patentansprüche

1. Kosmetische Zusammensetzung, umfassend von Iris-Rhizomen abgeleitete Exosomen, wobei die von Iris-Rhizomen abgeleiteten Exosomen mit einem Verfahren gereinigt werden, das die folgenden Schritte umfasst:
(A) Behandeln von Iris-Rhizomen mit einem Ultrahochdruck;
(B) Entsaften der mit dem Ultrahochdruck behandelten Iris-Rhizome;
(C) Gewinnen eines Überstands durch Zentrifugieren eines Saftes der Iris-Rhizome bei 1000 x g bis 10000 x g;
(D) Gefriertrocknen des Exosomen enthaltenden Überstands;
(E) Bilden eines wässrigen Zweiphasensystems durch Aufbringen von PEG (Polyethylenglykol)/Dextran auf das gefriergetrocknete Produkt; und
(F) Gewinnen einer Lösung der unteren Schicht, in der die Exosomen unterhalb des wässrigen Zweiphasensystem konzentriert sind,
wobei die Behandlung mit Ultrahochdruck mit einem Druck von 200 bis 500 MPa bei einer Temperatur von 15 bis 25 °C über 20 Sekunden bis 2 Minuten durchgeführt wird,
wobei die kosmetische Zusammensetzung weiterhin mindestens eines, ausgewählt aus der Gruppe bestehend aus Polyethylenglykol, Polypropylenglykol, einem Copolymer von Polyethylenglykol und Polypropylenglykol oder einem Derivat davon, Butylenglykol, Propylenglykol und Glycerin, enthält, um eine Stabilität der Eigenschaften der von Iris-Rhizomen abgeleiteten Exosomen zu verbessern.

2. Kosmetische Zusammensetzung nach Anspruch 1, **dadurch gekennzeichnet, dass** die von Iris-Rhizomen abgeleiteten Exosomen in einer Menge von 0,0001 bis 30,0 Gew.-% (w/w), bezogen auf das Gesamtgewicht der kosmetischen Zusammensetzung, enthalten sind.

3. Kosmetische Zusammensetzung nach Anspruch 1, **dadurch gekennzeichnet, dass** sie zur Befeuchtung einer Haut dient.

4. Kosmetische Zusammensetzung nach Anspruch 1, **dadurch gekennzeichnet, dass** sie zur Verbesserung von Hautfalten dient.

5. Kosmetische Zusammensetzung nach Anspruch 1, **dadurch gekennzeichnet, dass** sie zur Aufhellung einer Haut dient.

## Revendications

1. Composition cosmétique comprenant des exosomes dérivés du rhizome de l'iris, dans laquelle les exosomes dérivés du rhizome de l'iris sont purifiés par un procédé comprenant les étapes consistant à :
(A) traiter des rhizomes d'iris à une ultra-haute pression ;
(B) tirer le jus des rhizomes d'iris traités à l'ultra-haute pression ;
(C) obtenir un surnageant en centrifugeant un jus des rhizomes d'iris à 1 000 xg à 10 000 xg ;
(D) lyophiliser le surnageant contenant des exosomes ;
(E) former un système à deux phases aqueuses en appliquant du PEG (polyéthylène glycol)/dextrane au produit lyophilisé ; et
(F) obtenir une solution à couche inférieure dans laquelle les exosomes sont concentrés sous le système à deux phases aqueuses,
dans laquelle le traitement à ultra-haute pression est réalisé à une pression de 200 à 500 Mpa à une température comprise entre 15 et 25 °C pendant 20 secondes à 2 minutes,
dans laquelle la composition cosmétique contient en outre au moins un sélectionné dans le groupe constitué de polyéthylène glycol, de polypropylène glycol, d'un copolymère de polyéthylène glycol et de polypropylène glycol ou d'un dérivé de celui-ci, de butylène glycol, de propylène glycol et de glycérine, de façon à augmenter une stabilité de propriété des exosomes dérivés de rhizome d'iris.

2. Composition cosmétique selon la revendication 1, **caractérisée en ce que** les exosomes dérivés du rhizome d'iris sont compris dans une quantité de 0,0001 à 30,0 % en poids (w/w) sur la base du poids total de la composition cosmétique.

3. Composition cosmétique selon la revendication 1, **caractérisée en ce qu'**elle est destinée à hydrater la peau.

4. Composition cosmétique selon la revendication 1, **caractérisée en ce qu'**elle est destinée à atténuer les rides de la peau.

5. Composition cosmétique selon la revendication 1, **caractérisée en ce qu'**elle est destinée à blanchir la peau.
